# EUROPÄISCHE PATENTSCHRIFT

(11) **EP 0 940 676 B1**
(45) Veröffentlichungstag und Bekanntmachung des Hinweises auf die Patenterteilung: **02.04.2003**
(21) Anmeldenummer: 99104140.1
(22) Anmeldetag: 02.03.1999
(51) Int. Cl.: G01N 30/60

(54) **Vorrichtung zur Behandlung von Biomolekülen**
Device for treating biomolecules
Dispositif pour traiter des molécules biologiques

(30) Priorität: 04.03.1998 DE 29803712 U
(43) Veröffentlichungstag der Anmeldung: 08.09.1999
(73) Patentinhaber: Macherey, Nagel GmbH & Co. Handelsgesellschaft, 52355 Düren (DE)
(72) Erfinder: Sieber, Manfred Dr., 52385 Niedeggen (DE); Zeidler, Robert Dr., 52372 Kreuzau-Drove (DE)
(74) Vertreter: Paul, Dieter-Alfred, Dipl.-Ing.

(56) Entgegenhaltungen:
- EP-A- 0 588 564
- DE-A- 4 208 732
- DE-U- 29 803 712
- US-A- 4 787 971
- US-A- 4 832 916
- US-A- 4 956 298

## Beschreibung

Die Erfindung betrifft eine Vorrichtung zur Behandlung von Biomolekülen, insbesondere zur Isolierung von Nucleinsäuren, mit einer Trennsäule, die einen obenseitigen Einlaß und einen untenseitigen Auslaß aufweist und in der eine Trenneinrichtung angeordnet ist, und mit einem Auffanggefäß zum Auffangen der aus dem Auslaß austretenden Flüssigkeit.

Der Nachweis von viralen Nucleinsäuren wird auf vielen Gebieten der molekularen Medizin bzw. Diagnostik immer wichtiger. Verbesserte Therapiestrategien machen es notwendig, den Virustiter (z.B. bei HIV) bis hin zu kleinsten Werten zu verfolgen. Blutproben und -spenden müssen hinsichtlich ihrer viralen Belastung untersucht werden, was dazu führt, daß ein sehr hohes Probenaufkommen entsteht. Um Aufwand und Probenanzahl zu reduzieren, werden Proben vereinigt, um zum Beispiel zehn Proben zusammen zu untersuchen. Dies macht es unter anderem notwendig, Vorrichtungen und Verfahren zu entwickeln, mit denen größere Probenvolumina bearbeitet und trotzdem auch kleine Virusbelastungen erfaßt werden können. Ein ähnliches Problem stellt sich bei der Isolierung von DNA-Mengen aus biologischen Flüssigkeiten, beispielsweise Urin, Liquor etc.

Weitere mögliche Anwendungen sind die spezifische Bindung und Isolierung von Proteinen oder Peptiden, die in niedrigen Konzentrationen in biologischen Matrizes, z.B. Serum vorkommen und nachgewiesen werden müssen.

Aus der EP 0 616 638 B1 und der DE 38 43 610 A1 sind Vorrichtungen bekannt, welche aus einer Trennsäule und einem Auffanggefäß bestehen. Die Trennsäule weist einen obenseitigen Einlaß für die Aufgabe der Probe und einen untenseitigen Auslaß, der in das Auffanggefäß hineinragt, auf. Im unteren Bereich der Trennsäule befindet sich eine Trenneinrichtung beispielsweise in Form von ein- oder mehrstufigen Filtern. Die Trenneinrichtung ist so ausgebildet, daß die zu gewinnenden Biomoleküle adsorptiv an der Trenneinrichtung gebunden werden. Soweit Inhaltsstoffe z. B. aus Zellmaterial gewonnen werden sollen, gehen dem gewöhnlich mehrere Schritte zum Aufschluß und zur Reinigung voraus. Nach der Isolierung werden die Biomoleküle mittels eines Eluats von der Trenneinrichtung abgelöst und in einem gesonderten Auffanggefäß gesammelt. Sie stehen dann für weitere Untersuchungen zur Verfügung.

Bei den bekannten Vorrichtungen sind relativ große Elutionsvolumina erforderlich, um die isolierten Biomoleküle herauszulösen. Entsprechend gering ist die Konzentration der Biomoleküle in dem Eluat. Da in den angeschlossenen Detektionsverfahren nur vergleichsweise geringe Volumina eingesetzt werden können, macht es erhebliche Schwierigkeiten, kleine Mengen von Biomolekülen, wie Nukleinsäuren zu analysieren.

Es sind deshalb Versuche unternommen worden, mit Hilfe von Ultrazentrifugation bzw. Ultrafiltration eine Aufkonzentrierung vorzunehmen. Diese Verfahren haben sich jedoch nicht besonders bewährt, da sie weniger reproduzierbar sind und zudem einen erheblichen Geräte- und Zeitaufwand benötigen.

Der Erfindung liegt die Aufgabe zugrunde, eine Vorrichtung der eingangs genannten Art so auszubilden, daß man ohne separaten Aufkonzentrierungsschritt auch bei großen Probenvolumina mit einem wesentlich geringeren Elutionsvolumen auskommt. Gleichzeitig sollen Kreuzkontaminationen sicher vermieden werden.

Diese Aufgabe wird erfindungsgemäß dadurch gelöst, daß das Verhältnis von Innenvolumen der Trennsäule oberhalb der Trenneinrichtung zur Oberfläche der Trenneinrichtung mindestens 10 ml/cm², vorzugsweise mindestens 30 ml/cm² beträgt, und daß ein Außengefäß vorhanden ist, in das die Trennsäule und das damit verbundene Auffanggefäß einsteckbar sind. Hierbei kann es sich um ein übliches Zentrifugiergefäß mit obenseitiger Öffnung und einem Absatz für die Aufnahme in einer Zentrifuge handeln. Es hat sich gezeigt, daß bei dieser Geometrie der Trennsäule wesentliche geringere Elutionsvolumina erforderlich sind, um die aufgereinigten Biomoleküle aus der Trenneinrichtung herauszulösen. Das Volumenverhältnis zwischen Probe und Eluat liegt mindestens bei 8 und kann bis zu 100 gehen. Es wird also eine Aufkonzentration der Biomoleküle im Eluat erzielt, die vorherige oder nachträgliche Aufkonzentrierungsschritte selbst bei geringer Biomolekülkonzentration in der Probe erübrigt und die anschließende Analyse wesentlich erleichtert und zuverlässiger macht. Das Außengefäß bietet eine hohe Sicherheit gegen Kreuzkontamination, und es dient zudem als Auffanggefäß bei der Bindung der Biomoleküle aus der Probe an die Trenneinrichtung und bei nachfolgenden Waschschritten. Hierbei fallen vergleichsweise große Volumina an, welche von dem Außengefäß aufgenommen werden können. Für die Elution der aufgereinigten Biomoleküle kann dann aufgrund der erfindungsgemäßen Geometrie und der hieraus folgenden geringen Eluatmenge ein vergleichsweise kleines Auffanggefäß verwendet werden.

In Ausbildung der Erfindung ist vorgesehen, daß die Oberfläche der Trenneinrichtung unter 0,4 cm², vorzugsweise unter 0,3 cm² liegt, und zwar bei einem Innenvolumen von mindestens 15 ml, vorzugsweise 20 ml.

Grundsätzlich läßt sich die erfindungsgemäß Geometrie auch mit zylindrischen Trennsäulen erzielen. Damit die Trennsäule kompakt ist, ist es jedoch zweckmäßiger, daß sich der innere Querschnitt der Trennsäule von der Trenneinrichtung zum Einlaß hin beispielsweise unter Ausformung eines Trichterabschnittes vergrößert. Dabei können sich oben- und/oder untenseitig an den Trichterabschnitt jeweils Zylinderabschnitte anschließen. Die Trenneinrichtung sollte sich auf einem Absatz abstützen, durch den sich der Querschnitt des Auslasses verjüngt.

In besonders bevorzugter Ausbildung weist die Vorrichtung ein Außengefäß auf, in den die Trennsäule und das damit verbundene Auffanggefäß einsteckbar sind. Hierbei kann es sich um ein übliches Zentrifugiergefäß mit obenseitiger Öffnung und einem Absatz für die Aufnahme in einer Zentrifuge handeln. Das Außengefäß bietet eine hohe Sicherheit gegen Kreuzkontamination, und es dient zudem als Auffanggefäß bei der Bindung der Biomoleküle aus der Probe an die Trenneinrichtung und bei nachfolgenden Waschschritten. Hierbei fallen vergleichsweise große Volumina an, welche von dem Außengefäß aufgenommen werden können. Für die Elution der aufgereinigten Biomoleküle kann dann aufgrund der erfindungsgemäßen Geometrie und der hieraus folgenden geringen Eluatmenge ein vergleichsweise kleines Auffanggefäß verwendet werden. Vorzugsweise ist das Außengefäß so dimensioniert, daß die Trennsäule und das damit verbundene Auffanggefäß im wesentlichen vollständig in das Außengefäß passen. Dabei sollte das Außengefäß eine Länge haben, die der Länge der Trennsäule und des damit verbundenen Auffanggefäßes entspricht und beide im Außengefäß unbeweglich gehalten sind, so daß es bei der Zentrifugation nicht zu Lageveränderungen kommt.

Das Außengefäß sollte zweckmäßigerweise einen Verschlußdeckel aufweisen, um Trennsäule und Auffanggefäß vollständig einzuschließen und somit eine Barriere gegen Kreuzkontamination zu bilden. um eine Eigenbewegung der Trennsäule zu vermeiden, ist es ferner von Vorteil, wenn die Trennsäule und das damit verbundene Auffanggefäß zwischen Verschlußdeckel und Boden des Außengefäßes verspannbar ist. Dem selben Zweck dient es, wenn die Trennsäule einen den oberen Rand des Außengefäßes übergreifenden Ringsteg aufweist, der zwischen Verschlußdeckel und Außengefäß eingespannt werden kann und damit als Abdichtung fungiert.

Nach der Erfindung ist ferner vorgesehen, daß das Auffanggefäß luftdicht mit der Trennsäule verbindbar ist, um einen Austritt der aufgefangenen Substanz zu vermeiden. Das Auffanggefäß kann auf den Auslaß der Trennsäule aufsteckbar ausgebildet sein, wobei hierzu auch ein Adapter zwischengesetzt werden kann.

Es versteht sich, daß das Auffanggefäß über einen Deckel verschließbar sein soll, damit das die Biomoleküle enthaltene Eluat sicher transportiert und gelagert werden kann. Hierzu kann der Deckel in an sich bekannter Weise über ein Band mit dem Auffanggefäß verbunden sein.

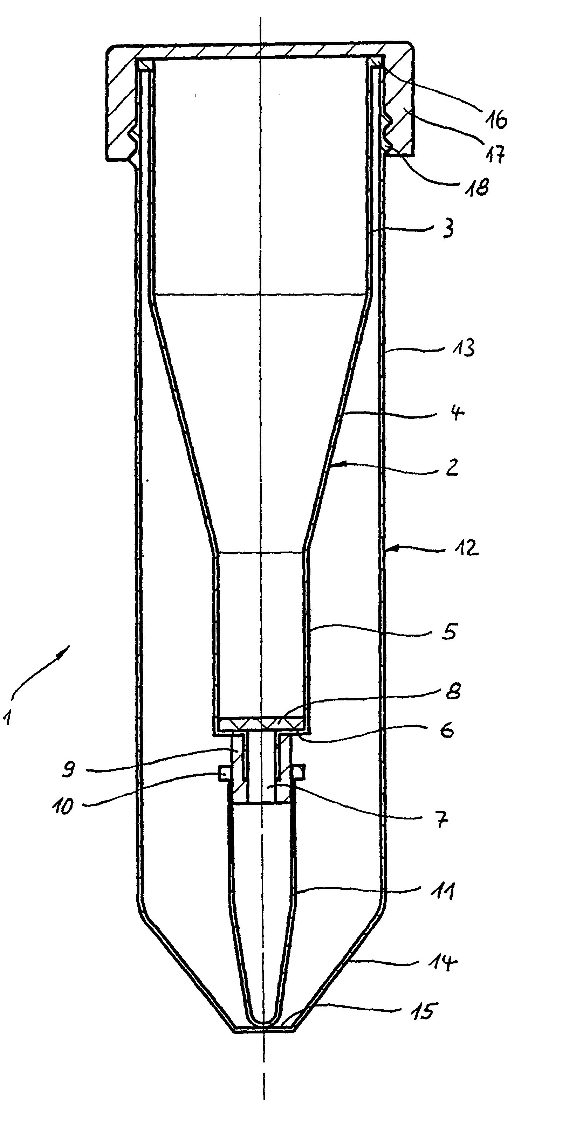

In der Zeichnung ist die Erfindung an Hand eines Ausführungsbeispiels näher veranschaulicht. Es zeigt eine Vorrichtung 1 zur Isolierung von viralen Nucleinsäuren.

Die Vorrichtung 1 weist eine Trennsäule 2 aus einem Kunststoffmaterial auf, welche einen obenseitigen Zylinderabschnitt 3, einen daran anschließenden, kegelstumpfförmigen Trichterabschnitt 4 und einen unteren Zylinderabschnitt 5 hat, welcher in einem Ringabsatz 6 ausläuft, an den sich ein zylindrischer Auslaß 7 anschließt.

Auf dem Ringabsatz 6 ruht eine Filterscheibe 8, die entsprechend den zu isolierenden Biomolekülen ausgebildet ist. Hierfür kommen beispielsweise die in der EP 0 616 638 B1 offenbarten Filter in Frage.

Auf den Auslaß 7 ist eine Adapterhülse 9 aufgesteckt. Auf die Adapterhülse 9 ist ein kleinvolumiges mit einem oberen Rand 10 versehenes Auffanggefäß 11 aufgesetzt, wobei die Anlage der Innenseite des Auffangefäßes 11 an der Außenseite der Adapterhülse 9 luftdicht ist.

Die Einheit aus Trennsäule 2 und Auffanggefäß 11 ist in ein Zentrifugiergefäß 12 eingesetzt. Das Zentrifugiergefäß 12 hat im wesentlichen eine Zylinderwandung 13, welche untenseitig in einem Kegelabschnitt 14 mit Boden 15 ausläuft. Auf dem Boden 15 stützt sich das Auffangefäß 11 ab. Auf dem oberen Rand des Auffanggefäßes 11 sitzt ein Ringsteg 16, der den oberen Rand der Trennsäule 2 bildet. Die Oberseiten von Zentrifugiergefäß 12 und Trennsäule 2 sind durch einen hutförmigen Verschlußdeckel 17 verschlossen, der den Ringsteg 16 einklemmt und über ein Gewinde 18 mit dem Zentrifugiergefäß 12 verbunden ist.

Mit der vorbeschriebenen Vorrichtung 1 können virale Nucleinsäuren wie folgt gewonnen werden.

Zwei Milliliter einer Serum- oder Plasmaprobe werden mit einem Mehrfachen (Üblicherweise 2-4 x) Volumen an Lysispuffer LP1 (hier: 4 ml, LP1: 4.5 M Guanidinthiocyanat, 20 mM Tris/HCl pH 6.5, 20 mM EDTA pH 8,0, 1 % Triton X100) versetzt. Die Mischung wird mehrmals auf- und abpipettiert, gevortext und für mehrere Minuten bei Raumtemparatur inkubiert. Wenn die Lysismischung sichtbare Partikel und/oder Trübungen aufweist, muß diese für 5-10 min zentrifugiert werden, um Feststoffe, die eventuell die Membran verstopfen können, abzutrennen. Wenn sehr kleine Mengen Nukleinsäure zu erwarten sind, muß dem Lysispuffer zur Erhöhung der Ausbeute unspezifische RNA ("carrier RNA": z. Bsp. poly(A)) zugesetzt werden.

Zu dem klaren Lysat wird vorteilhafterweise eine vergleichbare Menge (Endkonzentration 5-50 %) Ethanol (>98%), Isopropanol oder Aceton zugegeben (hier: 4 ml) und erneut durch Vortexen gründlich gemischt. Danach kann die Mischung in die Trennsäule 2 eingefüllt werden. Die Trennsäule 2 wird dann in ein 50 ml Zentrifugiergefäß 12 eingesetzt. Die Probe wird nachfolgend für 3 min bei einer Umdrehungsgeschwindigkeit von 1500 rpm zentrifugiert. Die Lösung passiert die Filterscheibe 8, wobei Nukleinsäuren binden. Der Durchlauf wird im Zentrifugiergefäß 12 gesammelt.

Die Trennsäule 2 wird anschließend mit mehreren Millilitern Waschpuffer WP1 (hier: 3 ml, WP1: 80% 1.5 M GTC, 30 mM Natriumcitrat pH 7.2, 20% Ethanol) befüllt. Der Zentrifugationsschritt wird wie oben beschrieben wiederholt und der Durchfluß verworfen. Durch die Waschschritte mit Ethanol-haltigem WP1 und anderen Waschpuffern werden vor allem unerwünschte Begleitstoffe von der Filterscheibe 8 entfernt, während die viralen Nukleinsäuren gebunden bleiben.

Die Trennsäule 2 wird jetzt mit Waschpuffer WP2 (WP2: 20 mM Tris/HCl pH 7.5, 50 mM NaCl, 80 % Ethanol) befüllt. Der Zentrifugationsschritt wird wie oben beschrieben durchgeführt, der Durchfluß verworfen und die Waschprozedur noch einmal auf die gleiche Weise wiederholt. Durch die Waschschritte mit stark Ethanol-haltigem Waschpuffer WP2 werden vor allem die chaotropen Salze des Lysispuffer von der Filterscheibe 8 abgewaschen.

Vor der Elution kann optional ein weiterer Waschschritt mit Aceton durchgeführt werden, um Ethanolspuren zu entfernen. Manche nachfolgend ausgeführten Amplifikationsprozeduren beinhalten Ethanol-sensitive Enzyme, so daß die Detektion viraler Nukleinsäuren in Anwesenheit von Ethanol inhibiert, wenn nicht ganz verhindert wird.

Danach wird die Trennsäule 2 leer weitere 10 min bei 3000-5000 rpm zentrifugiert, um Waschpufferreste bzw. Acetonreste zu entfernen. Zum vollständigen Austrocknen kann die Trennsäule 2 dann noch 5-10 min bei Raumtemperatur oder 37°C aufbewahrt werden.

Vor der Elution wird die Trennsäule 2 in ein frisches, steriles 50 ml Zentrifugiergefäß 12 überführt, wobei zunächst mittels der Adapterhülse 9 am unteren Auslauf 7 ein 500 µl Auffanggefäß 11 luftdicht aufgesteckt wurde. Jetzt werden 100 µl RNase-freies Wasser (60°C vortemperiert) direkt auf die Filterscheibe 8 pipetiert und für 1 min inkubiert. Durch 3 min Zentrifugation bei 3000 rpm wird die virale Nukleinsäuren enthaltende Elutionsfraktion in das unter der Trennsäule befindliche Auffanggefäß 11 überführt. Dieses wird zur Aufbewahrung der Probe von der Trennsäule 2 abgezogen und mit einem Deckel (hier nicht dargestellt) verschlossen.

## Patentansprüche

1. Vorrichtung (1) zur Behandlung von Biomolekülen, insbesondere zur Isolierung von Nucleinsäuren, mit einer Trennsäule (2), die einen obenseitigen Einlaß und einen untenseitigen Auslaß (7) aufweist und in der eine Trenneinrichtung (8) angeordnet ist, und mit einem Auffanggefäß (11) zum Auffangen der aus dem Auslaß (7) austretenden Flüssigkeit, **dadurch gekennzeichnet, daß** das Verhältnis von Innenvolumen der Trennsäule (2) oberhalb der Trenneinrichtung (8) zur Oberfläche der Trenneinrichtung (8) mindestens 10 ml/cm² beträgt und daß ein Außengefäß (12) vorhanden ist, in das die Trennsäule (2) und das damit verbundene Auffanggefäß (11) einsteckbar sind.

2. Vorrichtung nach Anspruch 1, **dadurch gekennzeichnet, daß** das Verhältnis mindestens 30 ml/cm² beträgt, vorzugsweise über 70 ml/cm².

3. Vorrichtung nach Anspruch 1 oder 2, **dadurch gekennzeichnet, daß** die Oberfläche der Trenneinrichtung (8) unter 0,4 cm² liegt, vorzugsweise unter 0,3 cm².

4. Vorrichtung nach einem der Ansprüche 1 bis 3, **dadurch gekennzeichnet, daß** das Innenvolumen der Trennsäule (2) mindestens 15 ml, vorzugsweise mindestens 20 ml beträgt.

5. Vorrichtung nach einem der Ansprüche 1 bis 4, **dadurch gekennzeichnet, daß** sich der innere Querschnitt der Trennsäule (2) von der Trenneinrichtung (8) zum Einlaß hin vergrößert.

6. Vorrichtung nach Anspruch 5, **dadurch gekennzeichnet, daß** die Trennsäule (2) zwischen Einlaß und Trenneinrichtung (8) einen Trichterabschnitt (4) aufweist.

7. Vorrichtung nach Anspruch 6, **dadurch gekennzeichnet, daß** sich an den Trichterabschnitt (4) oben - und/oder untenseitig jeweils ein Zylinderabschnitt (3, 5) anschließt.

8. Vorrichtung nach einem der Ansprüche 1 bis 7, **dadurch gekennzeichnet, daß** sich die Trenneinrichtung (8) auf einen Absatz (6) abstützt, durch den sich der Querschnitt des Auslasses (7) verjüngt.

9. vorrichtung nach einem der Ansprüche 1 bis 8, **dadurch gekennzeichnet, daß** die Trennsäule (2) und das damit verbundene Auffanggefäß (11) im wesentlichen vollständig in das Außengefäß (12) passen.

10. Vorrichtung nach Anspruch 9, **dadurch gekennzeichnet, daß** das Außengefäß (12) eine Länge hat, die der Länge der Trennsäule (2) und des damit verbundenen Auffanggefäßes (11) entspricht.

11. Vorrichtung nach Anspruch 9 oder 10, **dadurch gekennzeichnet, daß** die Trennsäule (2) und das damit verbundene Auffanggefäß (11) unbeweglich in dem Außengefäß (12) gehalten sind.

12. Vorrichtung nach einem der Ansprüche 1 bis 11, **dadurch gekennzeichnet, daß** das Außengefäß (12) einen Verschlußdeckel (17) aufweist.

13. Vorrichtung nach Anspruch 12, **dadurch gekennzeichnet, daß** der Verschlußdeckel (17) hutförmig ausgebildet ist, wobei ein Ringsteg als Überstand den oberen Endbereich des Außengefäßes (12) überdeckt.

14. Vorrichtung nach einem der Ansprüche 1 bis 13, **dadurch gekennzeichnet, daß** die Trennsäule (2) und das damit verbundene Auffanggefäß (11) zwischen verschlußdeckel (17) und Boden (15) des Außengefäßes (12) verspannbar ist.

15. Vorrichtung nach einem der Ansprüche 1 bis 14, **dadurch gekennzeichnet, daß** die Trennsäule (2) einen den oberen Rand des Außengefäßes (12) übergreifenden Ringsteg (16) aufweist.

16. Vorrichtung nach einem der Ansprüche 1 bis 15, **dadurch gekennzeichnet, daß** das Auffanggefäß (11) luftdicht mit der Trennsäule (2) verbindbar ist.

17. Vorrichtung nach einem der Ansprüche 1 bis 16, **dadurch gekennzeichnet, daß** das Auffanggefäß (11) auf den Auslaß (7) der Trennsäule (2) aufsteckbar ist.

18. Vorrichtung nach Anspruch 17, **dadurch gekennzeichnet, daß** auf den Auslaß (7) der Trennsäule (2) ein Adapter (9) aufgesetzt ist, der zu dem Auffanggefäß (11) paßt.

19. Vorrichtung nach einem der Ansprüche 1 bis 18, **dadurch gekennzeichnet, daß** das Auffanggefäß (11) über einen Deckel verschließbar ist.

20. Vorrichtung nach Anspruch 19, **dadurch gekennzeichnet, daß** der Deckel über ein Band mit dem Auffanggefäß (11) verbunden ist.

## Claims

1. Device (1) for treating biomolecules, in particular for isolating nucleic acids, having a separating column (2), which exhibits an inlet at the top and an outlet (7) at the bottom and in which a separating facility (8) is arranged, and having a collecting vessel (11) for collecting the liquid which issues from the outlet (7), **characterized in that** the ratio of the internal volume of the separating column (2) above the separating facility (8) to the area of the separating facility (8) is at least 10 ml/cm² and **in that** an outer vessel (12), into which the separating column (2) and the collecting vessel (11) which is connected to it can be inserted, is present.

2. Device according to Claim 1, **characterized in that** the ratio is at least 30 ml/cm², preferably more than 70 ml/cm².

3. Device according to Claim 1 or 2, **characterized in that** the area of the separating facility (8) is less than 0.4 cm², preferably less than 0.3 cm².

4. Device according to one of Claims 1 to 3, **characterized in that** the internal volume of the separating column (2) is at least 15 ml, preferably at least 20 ml.

5. Device according to one of Claims 1 to 4, **characterized in that** the internal sectional area of the separating column (2) increases from the separating facility (8) in the direction towards the inlet.

6. Device according to Claim 5, **characterized in that** the separating column (2) exhibits a funnel section (4) between the inlet and the separating facility (8).

7. Device according to Claim 6, **characterized in that** a cylindrical section (3, 5) in each case adjoins the funnel section (4) at the top and/or at the bottom.

8. Device according to one of Claims 1 to 7, **characterized in that** the separating facility (8) is supported on a shoulder (6) by which the sectional area of the outlet (7) is reduced.

9. Device according to one of Claims 1 to 8, **characterized in that** the separating column (2) and the collecting vessel (11) which is connected to it essentially fit completely into the outer vessel (12).

10. Device according to Claim 9, **characterized in that** the length of the outer vessel (12) corresponds to the length of the separating column (2) and the collecting vessel (11) which is connected to it.

11. Device according to Claim 9 or 10, **characterized in that** the separating column (2) and the collecting vessel (11) which is connected to it are held rigidly in the outer vessel (12).

12. Device according to one of Claims 1 to 11, **characterized in that** the outer vessel (12) exhibits a closure cap (17).

13. Device according to Claim 12, **characterized in that** the closure cap (17) is formed in the shape of a hat, with an annular ridge overlapping, as a projection, the upper end region of the outer vessel (12).

14. Device according to one of Claims 1 to 13, **characterized in that** the separating column (2) and the collecting vessel (11) which is connected to it can be braced between the closure cap (17) and the bottom (15) of the outer vessel (12).

15. Device according to one of Claims 1 to 14, **characterized in that** the separating column (2) exhibits an annular ridge (16) which overlaps the upper rim of the outer vessel (12).

16. Device according to one of Claims 1 to 15, **characterized in that** the collecting vessel (11) can be connected to the separating column (2) in an airtight manner.

17. Device according to one of Claims 1 to 16, **characterized in that** the collecting vessel (11) can be mounted on the outlet (7) of the separating column (2).

18. Device according to Claim 17, **characterized in that** an adapter which fits the collecting vessel (11) is attached to the outlet (7) of the separating column (2).

19. Device according to one of Claims 1 to 18, **characterized in that** the collecting vessel (11) can be closed with a lid.

20. Device according to Claim 19, **characterized in that** the lid is connected to the collecting vessel (11) by means of a tape.

## Revendications

1. Dispositif (1) pour le traitement de biomolécules, en particulier pour l'isolement d'acides nucléiques, avec une colonne de fractionnement (2) qui présente une entrée supérieure et une sortie inférieure (7) et dans laquelle se trouve un dispositif de séparation (8), et avec un récipient collecteur (11) pour la réception du liquide s'écoulant de la sortie (7), **caractérisé par le fait que** le rapport entre le volume intérieur de la colonne de fractionnement (2) au-dessus du dispositif de séparation (8) et la surface du dispositif de séparation (8) est d'au moins 10 ml/cm² et par la présence d'un récipient extérieur (12) dans lequel la colonne de fractionnement (2) et le récipient collecteur (11) associé sont emboîtables.

2. Dispositif selon la revendication 1, **caractérisé par le fait que** le rapport est d'au moins 30 ml/cm², de préférence supérieur à 70 ml/cm².

3. Dispositif selon la revendication 1 ou 2, **caractérisé par le fait que** la surface du dispositif de séparation (8) est inférieure à 0,4 cm², de préférence inférieure à 0,3 cm².

4. Dispositif selon l'une des revendications 1 à 3, **caractérisé par le fait que** le volume intérieur de la colonne de fractionnement (2) est d'au moins 15 ml, de préférence au moins 20 ml.

5. Dispositif selon l'une des revendications 1 à 4, **caractérisé par le fait que** la section intérieure de la colonne de fractionnement (2) s'agrandit du dispositif de séparation (8) vers l'entrée.

6. Dispositif selon la revendication 5, **caractérisé par le fait que** la colonne de fractionnement (2) présente un tronçon en forme d'entonnoir (4) entre l'entrée et le dispositif de séparation (8).

7. Dispositif selon la revendication 6, **caractérisé par le fait qu'**au tronçon en forme d'entonnoir (4) se raccordent des tronçons cylindriques (3, 5) respectivement supérieur et/ou inférieur.

8. Dispositif selon l'une des revendications 1 à 7, **caractérisé par le fait que** le dispositif de séparation (8) repose sur un palier (6) par lequel la section de la sortie (7) s'amincit.

9. Dispositif selon l'une des revendications 1 à 8, **caractérisé par le fait que** la colonne de fractionnement (2) et le récipient collecteur (11) associé tiennent pour l'essentiel entièrement dans le récipient extérieur (12).

10. Dispositif selon la revendication 9, **caractérisé par le fait que** le récipient extérieur (12) a une longueur qui correspond à la longueur de la colonne de fractionnement (2) et du récipient collecteur (11) associé.

11. Dispositif selon la revendication 9 ou 10, **caractérisé par le fait que** la colonne de fractionnement (2) et le récipient collecteur (11) associé sont maintenus immobiles dans le récipient extérieur (12).

12. Dispositif selon l'une des revendications 1 à 11, **caractérisé par le fait que** le récipient extérieur (12) présente un couvercle de fermeture (17).

13. Dispositif selon la revendication 12, **caractérisé par le fait que** le couvercle de fermeture (17) est en forme de chapeau, une nervure annulaire recouvrant, en débordant dessus, la partie terminale supérieure du récipient extérieur (12).

14. Dispositif selon l'une des revendications 1 à 13, **caractérisé par le fait que** la colonne de fractionnement (2) et le récipient collecteur (11) associé peuvent être calés entre le couvercle de fermeture (17) et le fond (15) du récipient extérieur (12).

15. Dispositif selon l'une des revendications 1 à 14, **caractérisé par le fait que** la colonne de fractionnement (2) présente une nervure annulaire (16) recouvrant le bord supérieur du récipient extérieur (12).

16. Dispositif selon l'une des revendications 1 à 15, **caractérisé par le fait que** le récipient collecteur (11) peut être mis en communication imperméable à l'air avec la colonne de fractionnement (2).

17. Dispositif selon l'une des revendications 1 à 16, **caractérisé par le fait que** le récipient collecteur (11) peut être emboîté sur la sortie (7) de la colonne de fractionnement (2).

18. Dispositif selon la revendication 17, **caractérisé par le fait qu'**un adaptateur (9), qui est ajusté au récipient collecteur (11), est engagé sur la sortie (7) de la colonne de fractionnement (2).

19. Dispositif selon l'une des revendications 1 à 18, **caractérisé par le fait que** le récipient collecteur (11) peut être fermé par un couvercle.

20. Dispositif selon la revendication 19, **caractérisé par le fait que** le couvercle est raccordé au récipient collecteur (11) par un collier.
